# NEUE EUROPÄISCHE PATENTSCHRIFT

(11) **EP 2 967 616 B2**
(45) Veröffentlichungstag und Bekanntmachung des Hinweises auf die Entscheidung über den Einspruch: **28.02.2024**
(45) Hinweis auf die Patenterteilung: 03.10.2018
(21) Anmeldenummer: 14710521.7
(22) Anmeldetag: 10.03.2014
(51) Int. Cl.: A61B 17/225, A61B 6/04, A61B 6/00

(54) **THERAPIESYSTEM**
THERAPY SYSTEM
SYSTÈME THÉRAPEUTIQUE

(30) Priorität: 14.03.2013 DE 102013204493
(43) Veröffentlichungstag der Anmeldung: 20.01.2016
(73) Patentinhaber: Richard Wolf GmbH, 75438 Knittlingen (DE)
(72) Erfinder: BURKHARDT, Michael, 75417 Mühlacker (DE); MESSINA, Francesco, 76703 Kraichtal (DE); ZIMMERMANN, Jean-Georges, 75438 Knittlingen (DE)
(74) Vertreter: Patentanwälte Hemmer Lindfeld Frese Partnerschaft mbB
(86) Internationale Anmeldenummer: PCT/EP2014/054580
(87) Internationale Veröffentlichungsnummer: WO 2014/139950

(56) Entgegenhaltungen:
- EP-A1- 0 269 801
- EP-A1- 2 308 398
- WO-A1-2011/061645
- DE-A1- 4 329 167
- DE-C1- 3 426 398
- US-A1- 2003 149 352
- US-A1- 2008 146 908
- US-A1- 2011 166 450
- US-A1- 2011 166 450

## Beschreibung

Die Erfindung betrifft ein Therapiesystem mit einer Strahlung oder Wellen aussendenden Therapiequelle mit den im Oberbegriff des Anspruchs 1 angegebenen Merkmalen sowie ein Verfahren zur Positionierung einer Patientenliege relativ zu einem Therapiekopf eines solchen Therapiesystems.

Bei Stoßwellen und Ultraschalltherapiesystemen, insbesondere Lithotriptoren ist es erforderlich, den Fokus der Therapiequelle definiert zum Patienten zu positionieren. Dazu ist es bekannt, den Patienten auf eine bewegbare Patientenliege zu legen, welche in drei Raumkoordinaten relativ zu der Therapiequelle bewegt werden kann. Dabei ist es bekannt, die Positionierung unter Zuhilfenahme von Röntgen- und/oder Ultraschallbildern vorzunehmen. Eine solche Vorrichtung ist beispielsweise aus EP 2 308 398 A1 bekannt.

Aus dieser Druckschrift ist es bereits bekannt, dass an der Bilddarstellung des Ultraschall- oder Röntgenbildes Positioniertasten angeordnet sind, welche es ermöglichen, den Patienten so zu verschieben, dass der Therapiefokus im Bild bewegt wird. Nachteilig bei diesen Anordnungen ist jedoch, dass aufgrund der zur Positionierung erforderlichen Verfahrbewegung entlang der drei Raumkoordinaten es häufig vorkommt, dass der Arzt den zu behandelnden Punkt, beispielsweise einen Stein aus dem aufgenommenen Ultraschallbild verliert. Dies macht die Positionierung sehr schwierig.

Es ist daher Aufgabe der Erfindung, ein derartiges Therapiesystem in der Weise zu verbessern, dass eine einfache und sichere Positionierung der Therapiequelle relativ zum Patienten unter Zuhilfenahme eines Ultraschallbildes möglich ist. Diese Aufgabe wird durch ein Therapiesystem mit den in Anspruch 1 angegebenen Merkmalen sowie durch ein Verfahren mit den in Anspruch 12 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße Therapiesystem weist eine Strahlung oder Wellen aussende Therapiequelle auf. Dies kann insbesondere eine Therapiequelle sein, welche Ultraschall oder Stoßwellen aussendet, d. h. beispielsweise ein Stoßwellenwandler. Das erfindungsgemäße Therapiesystem weist darüber hinaus eine Ultraschallsonde auf, mit deren Hilfe zu behandelnde Punkte im Patientenkörper, beispielsweise Steine wie Nieren- oder Gallensteine aufgefunden werden können. Ferner ist eine Patientenliege vorgesehen. Das Therapiesystem ist mit einem Mehr-Achsen-Positioniersystem mit mehreren Antrieben ausgestattet ist, mit dessen Hilfe die Patientenliege relativ zu der Therapiequelle in allen drei Raumrichtungen positioniert werden kann. Dazu kann die Patientenliege und/oder die Therapiequelle mittels des Positioniersystems bewegbar sein.

Erfindungsgemäß weist das Therapiesystem eine Steuereinrichtung auf, welche mit der Ultraschallsonde gekoppelt ist. Dabei ist die Kopplung insbesondere so ausgestaltet, dass die Steuereinrichtung die räumliche Lage des Ultraschallbildes erfasst. Ferner ist die Steuereinrichtung erfindungsgemöß so ausgebildet, dass sie in zumindest einem Betriebsmodus die Antriebe gleichzeitig, d. h. gemeinsam so ansteuert, dass die Patientenliege relativ zu der Therapiequelle entlang eines auswählbaren Bewegungspfades bewegt wird, welche innerhalb der Ebene eines aktuell von der Ultraschallsonde aufgenommenen Ultraschallbildes liegt. Das bedeutet, dass die Antriebe so verfahren werden, dass eine resultierende Bewegungsrichtung der Patientenliege relativ zu der Therapiequelle erzeugt wird, welche genau in der Bild- bzw. Schnittbildebene des Ultraschallbildes gelegen ist. D. h. hier werden nicht wie bei herkömmlichen Systemen, welche aus dem Stand der Technik bekannt sind, die Antriebe für die einzelnen Raumrichtungen separat nacheinander und unabhängig verfahren, sondern die Verfahrbewegung erfolgt in allen Raumrichtungen gleichzeitig, sodass ein resultierender Bewegungspfad geschaffen wird, welcher während der gesamten Bewegung in der Ebene des Ultraschallbildes liegt. Dazu berücksichtigt die Steuereinrichtung die Winkel und räumliche Lage des Ultraschallbildes. Durch ein solches System, welches eine Verfahrbewegung nicht in kartesischen Koordinaten sondern entlang von Vektoren realisiert, wird somit sichergestellt, dass es beim Bewegen des Patienten relativ zu der Therapiequelle nicht dazu kommt, dass der zu behandelnde Therapiepunkt, beispielsweise ein Stein, aus der Bildebene des Ultraschallbildes verfahren wird.

Das Mehr-Achsen-Positioniersystem kann entweder vollständig in die Patientenliege oder in die Therapiequelle integriert sein, d. h. entweder wird allein die Patientenliege oder allein die Therapiequelle bewegt. Alternativ ist es auch möglich, beide Elemente bewegbar auszugestalten, wobei die Bewegungsachsen der Therapiequelle und der Patientenliege dann so gewählt werden bzw. einander so ergänzen, dass zusammen eine Relativbewegung der Patientenliege zu der Therapiequelle in allen drei Raumrichtungen realisiert werden kann.

Die Steuereinrichtung ist so ausgebildet, dass sie erfindungsgemäß zumindest in einem Betriebsmodus den vorangehend geschilderten Betrieb zulässt. Das bedeutet, dass in der Steuereinrichtung noch weitere Betriebsmodi vorgesehen sein können, welche beispielsweise ein Verfahren der Patientenliege relativ zu der Therapiequelle in den drei Raumkoordinaten unabhängig zueinander ermöglichen.

Die Steuereinrichtung ist derart ausgebildet, dass sie in dem genannten zumindest einen Betriebsmodus die Antriebe so ansteuert, dass dieses zeitgleich mit individuell von der Steuereinrichtung definierten Geschwindigkeiten verfahren. Das bedeutet, dass beim zeitgleichen Betrieb der Antriebe diese nicht mit denselben Verfahrgeschwindigkeiten angetrieben werden müssen. Vielmehr werden von der Steuereinrichtung die Verfahrgeschwindigkeiten so definiert, dass die Verfahrbewegung entlang dem gewünschten Bewegungspfad in der Bildebene, d. h. der aktuellen Bildebene des Ultraschallbildes erreicht wird. So wird eine Verfahrbewegung entlang einer definierten Richtung oder Linie im Ultraschallbild erreicht, wobei eine Kontrolle im Ultraschallbild in Echtzeit möglich ist und sichergestellt wird, dass ein einmal gefundener Therapiepunkt, beispielsweise ein Stein bei der Bewegung die ganze Zeit im Ultraschallbild verbleibt.

Ferner ist die Steuereinrichtung derart ausgebildet, dass die Geschwindigkeiten, mit welchen die Antriebe verfahren werden, von der Steuereinrichtung derart festgelegt werden, dass das Verhältnis der Verfahrgeschwindigkeiten zueinander dem Verhältnis der von den Antrieben zum Verfahren entlang des Bewegungspfades zurückzulegenden Wege entspricht. D. h. vorzugsweise wird im Ultraschallbild zunächst der Bewegungspfad nach Richtung der Verfahrbewegung festgelegt. Bei gerader Bewegung wird somit ein Bewegungsvektor im Ultraschallbild definiert. Transformiert auf die drei Raumkoordinaten des Mehr-Achsen-Positioniersystems ergeben sich daraus für sämtliche Antriebe, d. h. jede Koordinatenachse mit einem Antrieb, bestimmte zurückzulegende Wege. Basierend auf diesen zurückzulegenden Wegen wird von der Steuereinrichtung die Verfahrgeschwindigkeit für die einzelnen Antriebe definiert. D.h. die Antriebe, welche einen weiteren Weg zurückzulegen haben als andere, werden entsprechend schneller bewegt. Das Verhältnis der zurückzulegenden Wege zueinander ergibt sich aus der jeweiligen Steigung des Bewegungspfades zu den Koordinatenachsen.

Zweckmäßigerweise ist die Steuereinrichtung derart ausgebildet, dass in dem Ultraschallbild als Bewegungspfad ein Vektor auswählbar ist, wie vorangehend beschrieben wurde. D. h. es wird ein gerader Bewegungspfad, in dem Ultraschallbild ausgewählt bzw. anhand des Ultraschallbildes vorgegeben. Dies kann beispielsweise durch entsprechende Betätigungstasten an einer Anzeigeeinrichtung für das Ultraschallbild geschehen.

In dem erfindungsgemäßen Therapiesystemen sind weiter bevorzugt drei Antriebe für das Mehr-Achsen-Positioniersystem vorgesehen, welche eine Bewegung entlang dreier zueinander normaler Achsen ermöglichen. Diese Achsen entsprechen somit den drei kartesischen Raumkoordinaten. So kann die Patientenliege hoch und runter sowie in Längsrichtung vor und zurück und in Querrichtung seitlich dazu verfahren werden. Erfindungswesentlich ist dabei, dass diese drei Antriebe stets zeitglich von der Steuereinrichtung so angesteuert werden, dass ihre Geschwindigkeiten und Wege so gewählt werden, dass die Verfahrbewegung entlang einem Bewegungspfad bzw. Vektor erfolgt, welcher in der Bildebene des Ultraschallbildes gelegen ist. Lediglich, wenn die Bildebene des Ultraschallbildes sich in nur zwei der drei Raumkoordinaten erstreckt, ist auch nur eine Verfahrbewegung der zwei zugehörigen Antriebe erforderlich, während der dritte Antrieb nicht angetrieben wird.

Die Ultraschallsonde ist vorzugsweise derart in die Therapiequelle integriert, dass ein Therapiefokus der Therapiequelle in dem von der Ultraschallsonde aufgenommenen Ultraschallbild gelegen ist. Der Therapiefokus ist dabei weiter bevorzugt in dem Ultraschallbild, beispielsweise durch ein Fadenkreuz gekennzeichnet. Durch entsprechende Bewegungstasten an der Anzeigeeinrichtung des Ultraschallbildes kann der Patient mit der Patientenliege relativ zu der Therapiequelle bewegbar sein, sodass ein zu behandelnden Punkt im Ultraschallbild, beispielsweise ein Stein zur Deckung mit dem Fadenkreuz gebracht werden kann. Die Antriebe des Mehr-Achsen-Positioniersystems werden dann dabei zeitgleich so verfahren, dass die Patientenliege relativ zu der Therapiequelle so bewegt wird, dass sie den Bewegungspfad des Koordinatenkreuzes im Ultraschallbild in dessen Bildebene folgt. D. h. der gefundene zu behandelnde Punkt wie ein Stein verbleibt während der gesamten Bewegung im Ultraschallbild. So lässt sich der gekennzeichnete Therapiefokus relativ leicht zu einem zu behandelnden Punkt bewegen.

Weiter bevorzugt ist die Ultraschallquelle um eine sich durch den Therapiefokus erstreckende Achse relativ zu der Therapiequelle drehbar, wobei ein mit der Steuereinrichtung verbundenes erstes Winkelmesssystem zum Erfassen des Drehwinkels zwischen der Ultraschallsonden und der Therapiequelle vorhanden ist. Durch die Drehung der Ultraschallsonde wird das Schnittbild, d. h. das aufgenommene Ultraschallbild um diese Achse gedreht. So kann im Ultraschallbild der zu behandelnde Punkt durch Drehung um diese Achse aufgefunden werden. Durch das erste Winkelmesssystem wird dabei die räumliche Lage, d. h. die Winkellage des Ultraschallbildes um diese Achse erfasst, sodass die Steuereinrichtung, wenn in diesem Ultraschallbild ein Bewegungspfad ausgewählt wird, diesen unter Berücksichtigung des Winkels räumlich genau bestimmen und insbesondere dem Koordinatensystem des Mehr-Achsen-Positioniersystems zuordnen kann, d. h. die Lage des Ultraschallbildes und damit des zu erreichenden Bewegungspfades in das Koordinatensystem des Mehr-Achsen-Positioniersystems übertragen kann.

Gemäß einer weiteren bevorzugten Ausführungsform ist die Therapiequelle relativ zu der Patientenliege entlang einer bogenförmigen Bahn schwenkbar, wobei ein mit der Steuereinrichtung verbundenes zweites Winkelmesssystem zum Erfassen des Schwenkwinkels zwischen der Therapiequelle und der Patientenliege vorhanden ist. Die Schwenkbarkeit entlang der bogenförmigen Bahn erfolgt vorzugsweise um eine Schwenkachse, welche sich parallel zur Längsachse der Patientenliege, d. h. parallel zur Longitudinalachse des auf der Patientenliege liegenden Patienten erstreckt. Da die Ultraschallsonde bevorzugt in die Therapiequelle integriert ist, verschwenkt sie entsprechend mit um diese bogenförmige Bahn. Durch das zweite Winkelmesssystem wird daher der Steuereinrichtung die Winkellage des Ultraschallbildes um diese zweite Schwenkachse bekannt gemacht, sodass bei der Definition eines Bewegungspfades in dem Ultraschallbild in Kenntnis dieses Winkels weiterhin der definierte Bewegungspfad, insbesondere Bewegungsvektor in das Koordinatensystem des Mehr-Achsen-Positioniersystems transformiert werden kann und entsprechend die Bewegung genau in der Bildebene des Ultraschallbildes erfolgen kann, unabhängig davon, in welche Richtung die Ultraschallsonde verschwenkt ist. D. h. die erfindungsgemäße Steuereinrichtung ermöglicht eine Verfahrbewegung der Patientenliege relativ zu der Therapiequelle entlang eines in dem Ultraschallbild gelegenen Pfades, unabhängig von der aktuellen räumlichen Lage des Ultraschallbildes.

Besonders bevorzugt ist die Steuereinrichtung derart ausgebildet, dass sie in dem zumindest einen Betriebsmodus die Antriebe derart ansteuert, dass eine Relativbewegung zwischen der Patientenliege und der Therapiequelle ausschließlich entlang von Bewegungspfaden möglich ist, welche innerhalb der Ebene des Ultraschallbildes gelegen ist. Dadurch wird verhindert, dass sich die Positionierung von Patient und Therapiequelle so ändert, dass ein gewünschter Therapiepunkt, beispielsweise ein Stein aus dem Ultraschallbild herausbewegt wird. So kann eine Fehlbedienung verhindert werden.

Weiter bevorzugt ist die Steuereinrichtung derart ausgebildet, dass von einem Benutzer der Bewegungspfad für eine gewünschte Relativbewegung zwischen Patientenliege und Therapiequelle innerhalb der Ebene des aktuellen Ultraschallbildes auswählbar ist. Dies kann beispielsweise durch Bewegung eines den Therapiefokus kennzeichnenden Fadenkreuzes im Ultraschallbild erfolgen. Alternativ können entsprechende Tasten zum Auslösen einer Bewegung am Monitor bzw. der Anzeigeeinrichtung für das Ultraschallbild angeordnet sein. Auch wäre es denkbar, über ein geeignetes Eingabemittel, beispielsweise eine Computermaus, einen Joystick (Steuerknüppel) oder einen Stift im Ultraschallbild eine Richtung oder Strecke zu definieren, entlang der eine Verfahrbewegung erfolgen soll.

Besonders bevorzugt sind auf einem das Ultraschallbild darstellenden Bildschirm oder seitlich einem das Ultraschallbild darstellenden Bildschirms Tasten zur Auswahl einer den Bewegungspfad definierenden Bewegungsrichtung angeordnet. So können insbesondere vier Tasten, welche den Bewegungsrichtungen oben, unten, rechts, links im Ultraschallbild entsprechend vorgesehen sein. Mit solchen Tasten kann eine Bewegung der Patientenliege relativ zu der Therapiequelle veranlasst werden, sodass sich ein Zielpunkt, z. B. ein Stein im Bild so verschiebt, dass er sich zu dem Therapiefokus bewegt, d. h. eine Bewegung zu der gewählten Lage des Therapiefokus entlang einer geraden Strecke, welche den Bewegungspfad definiert, erfolgt. Wenn der Bildschirm als berührungsempfindlicher Bildschirm ausgebildet ist, können solche Tasten virtuell in das Bild eingeblendet sein.

Die Steuereinrichtung ist zweckmäßigerweise derart ausgebildet, dass sie die räumliche Lage des ausgewählten Pfades in das Koordinatensystem des Mehr-Achsen-Positioniersystems transformiert und die Antriebe dann derart ansteuert, dass eine Bewegung entlang dem transformierten Pfad erfolgt. Dabei werden die Antriebe in der oben beschriebenen Weise zeitgleich mit definiert angepassten Geschwindigkeiten betrieben, sodass die Verfahrbewegung genau entlang dem Pfad, welcher in der aktuellen Bildebene des Ultraschallbildes gelegen ist, erfolgt.

Gemäß einer besonderen Ausführungsform der Erfindung kann ein Membrandruckregelsystem vorgesehen sein, welches derart ausgebildet ist, dass es ein Anlagedruck einer dem Patienten zugewandten Außenfläche der Therapiequelle durch Druckbeaufschlagung oder Druckentlastung einer in der Therapiequelle ausgebildeten Vorlaufstrecke konstant hält. Insbesondere bei Stoßwellenwandlern, welche eine Therapiequelle darstellen können, ist häufig eine flüssigkeitsgefüllte Vorlaufstrecke vorgesehen, welche zum Patienten hin durch eine Membran abgeschlossen ist. Um eine ausreichende Einkopplung der Stoßwellen in das Körpergewebe des Patienten zu ermöglichen, ist es erforderlich, dass die Membran in Anlage bzw. Kontakt mit der Haut des Patienten gehalten wird. Wenn der Patient nun durch das Mehr-Achsen-Positioniersystem oder Eigenbewegung näher zu der Therapiequelle hin oder von dieser wegbewegt wird, wäre eine solche Anlage bzw. ein solcher Kontakt möglicherweise nicht mehr sichergestellt. Durch das Membrandruckregelsystem wird jedoch die Vorlaufstrecke automatisch verlängert oder verkürzt, sodass die Membran des Therapiekopfes bzw. der Therapiequelle in definierter Anlage bzw. definiertem Kontakt an der Haut des Patienten gehalten wird. Das Membrandruckregelsystem kann beispielsweise eine Überwachung des Flüssigkeitsdruckes in der Vorlaufstrecke beinhalten, sodass dieser konstant oder in vordefinierten Grenzen gehalten wird. Dazu kann das Membrandruckregelsystem mit einer Pumpe gekoppelt sein, welche definiert zusätzlich Flüssigkeit in die Vorlaufstrecke pumpt oder aus der Vorlaufstrecke ablässt, um den Druck in der Vorlaufstrecke konstant zu halten. Die Flüssigkeit in der Vorlaufstrecke ist vorzugsweise Wasser. Anstelle einer Pumpe können zusätzlich oder alternativ Ventile vorgesehen sein, welche mit der Steuereinrichtung gekoppelt sind, um die Vorlaufstrecke definiert mit Flüssigkeit füllen oder definiert Flüssigkeit ablassen zu können, um den Druck im Inneren konstant zu halten. Bevorzugt ist entsprechend zumindest ein Drucksensor in der Therapiequelle vorhanden, welcher den Druck der Vorlaufstrecke erfassen kann und mit der Steuereinrichtung gekoppelt ist.

Neben dem beschriebenen erfindungsgemäßen Therapiesystem ist Gegenstand der Erfindung ein Verfahren zur Positionierung einer Patientenliege relativ zu einer Therapiequelle eines Therapiesystems, beispielsweise einen Stoßwellenwandler. Gemäß dem erfindungsgemäßen Verfahren wird die Therapiequelle anhand eines aktuellen Ultraschallbildes positioniert. Dazu wird in dem aktuellen Ultraschallbild in der Ebene des Ultraschallbildes ein Bewegungspfad, insbesondere ein Vektor definiert, entlang dem die Patientenliege relativ zu der Therapiequelle verfahren werden soll. Die Relativbewegung zwischen Patientenliege und Therapiequelle kann dabei entweder allein durch Bewegung der Patientenliege oder allein durch Bewegung der Therapiequelle oder aber auch durch gemeinsame Bewegung erreicht werden. Anschließend werden Antriebe, mittels welchen die Patientenliege relativ zu der Therapiequelle bewegbar ist, gleichzeitig so angetrieben, dass eine Bewegung der Patientenliege relativ zu dem Therapiekopf dem definierten Bewegungspfad folgt, d. h. die Bewegung folgt einem Bewegungspfad, welcher in der Bildebene des Ultraschallbildes gelegen ist. So erfolgt die Bewegung zu keinem Zeitpunkt aus dem Ultraschallbild heraus, sodass ein einmal im Ultraschallbild gefundener Therapiepunkt, beispielsweise ein Stein während der Verfahrbewegung im Ultraschallbild verbleibt und somit eine Kontrolle in Echtzeit möglich ist. Die Antriebe können, wie beschrieben, entweder allein die Patientenliege, die Therapiequelle oder aber beide bewegen, sodass eine Relativbewegung erreicht wird. Bezüglich der genauen Ausgestaltung wird die vorangehende Beschreibung des Therapiesystems verwiesen, aus welchem sich ebenfalls die wesentlichen Verfahrensmerkmale ergeben.

Zweckmäßigerweise wird die räumliche Lage des Bewegungspfades, welcher in dem Ultraschallbild ausgewählt wird, unter Berücksichtigung der aktuellen räumlichen Lage der Ebene des Ultraschallbildes in ein Koordinatensystem der Bewegungsachsen, entlang derer die Patientenliege relativ zu der Therapiequelle bewegt wird, transformiert und anschließend werden die Antrieben gleichzeitig so angesteuert, dass eine Bewegung entlang dieses transformierten Bewegungspfades erfolgt. Dabei werden Bewegungen in verschiedene Richtungen, d. h. entlang verschiedener Achsen nicht nacheinander sondern zeitgleich durchgeführt, wobei auch die Verfahrgeschwindigkeiten zweckmäßigerweise individuell für die einzelnen Achsen eingestellt werden. So erfolgt die Bewegung entlang einem Bewegungspfad, insbesondere einem Vektor, welcher in der Ebene des Ultraschallbildes gelegen ist.

Dazu werden die Verfahrgeschwindigkeiten der einzelnen Antriebe so eingestellt, dass deren Verhältnis zueinander dem Verhältnis der Wege zueinander entspricht, welche von den einzelnen Antrieben zum Verfahren entlang des Bewegungspfades zurückzulegen sind. Diese Wege entsprechen der Steigung des Vektors, welcher den Bewegungspfad definiert in der jeweiligen Raumrichtung des Koordinatensystems der Bewegungsachsen entlang derer die Patientenliege relativ zu der Therapiequelle bewegt wird. Die Antriebe, entlang deren Achse weitere bzw. längere Verfahrwege zurückzulegen sind, werden entsprechend im Verhältnis schneller verfahren.

Nachfolgend wird die Erfindung beispielhaft anhand der beigefügten Figuren beschrieben. In diesen zeigt:
- Fig. 1: schematisch die Gesamtanordnung eines erfindungsgemäßen Therapiesystems,
- Fig. 2: schematisch die Positionierung der Therapiequelle relativ zu einem Ultraschallbild,
- Fig. 3: schematisch das Ultraschallbild,
- Fig. 4: schematisch die Transformation der Bewegungskoordinaten aus dem Ultraschallbild und
- Fig. 5: eine schematische Gegenüberstellung der Bewegungsachsen im Ultraschallbild und der Therapiequelle relativ zur Patientenliege.

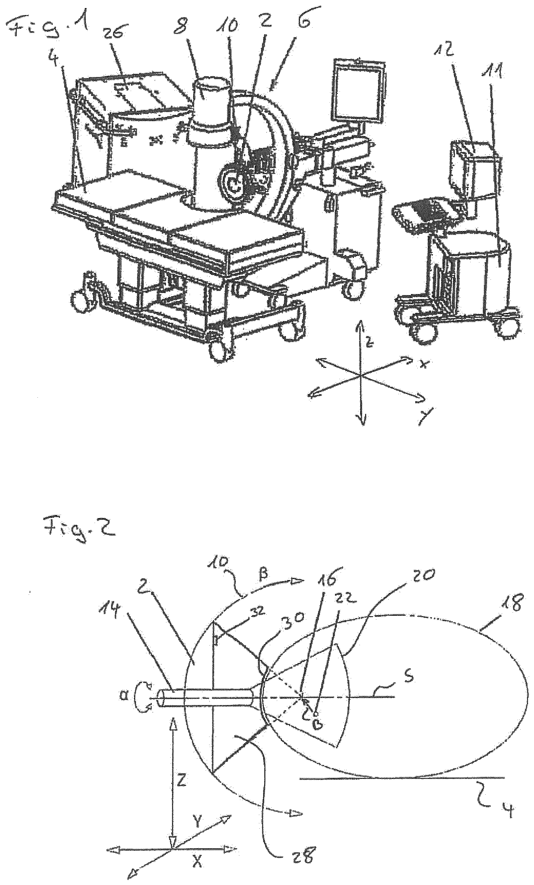

Fig. 1 zeigt beispielhaft ein Therapiesystem in Form einer Lithotripsieeinrichtung. Diese weist eine Therapiequelle in Form eines Stoßwellenwandlers auf. Ferner ist eine Patientenliege 4 vorgesehen, auf welcher ein zu behandelnder Patient zu liegen kommt und welche relativ zu der Therapiequelle 2 verfahr- und positionierbar ist. In diesem Ausführungsbeispiel ist ferner ein Röntgen-C-Bogen 6 mit einer Röntgenbildverstärker 8 vorgesehen. Die Therapiequelle 2 beinhaltet eine in Fig. 1 nicht einzeln gezeigte Ultraschallsonde und ist entlang einer bogenförmigen Führungsbahn 10 um eine Achse schwenkbar, welche sich parallel zur Längsachse der Patientenliege und somit zur Longitudinalachse eines Patienten auf der Patientenliege 4 erstreckt. Die Ultraschallsonde ist mit einem Ultraschallsteuergerät 11 verbunden, welches einen Ultraschallmonitor 12 aufweist.

Die Patientenliege 4 bzw. deren Oberfläche sind in zueinander rechtwinkligen Raumkoordinaten X, Y, und Z relativ zu der Therapiequelle 2 verfahrbar, um einen Patienten auf der Patientenliege 4 relativ zu der Therapiequelle positionieren zu können. Hierzu ist ein Mehr-Achsen-Positioniersystem vorgesehen, welches drei unabhängige Antriebe zum Realisieren der Verfahrbewegung entlang der Achsen X, Y, und Z aufweist. Die Antriebe sind hier nicht im Einzelnen gezeigt, können in üblicher Weise ausgebildet sein und werden hier durch die Achsen X, Y, und Z repräsentiert. Bei dem hier beschriebenen Ausführungsbeispiel ist vorgesehen, die Patientenliege mit den geschilderten Antrieben in Richtung der drei Achsen zu verfahren. Es ist jedoch zu verstehen, dass entsprechend auch die Therapiequelle 2 relativ zu der Patientenliege verfahren werden könnte. Auch wäre es denkbar, die Relativbewegung durch eine Überlagerung von Bewegungen der Patientenliege 4 und der Therapiequelle 2 zu realisieren. Die Steuerung der Antriebe erfolgt dabei entsprechend der nachfolgenden Beschreibung.

Wie schematisch in Fig. 2 gezeigt, ist die gesamte Therapiequelle 2 gemeinsam mit der in ihr angeordneten Ultraschallsonde 14 entlang der bogenförmigen Bahn 10 um den Winkel β schwenkbar. Die Ultraschallsonde 14 ist im Inneren der Therapiequelle 2 ferner um eine Achse S um den Winkel a drehbar. Die Achse S erstreckt sich dabei durch den Therapiefokus 16 der Therapiequelle 2.

Der Patient 18 kann über die Antriebe in den X-, Y-, und Z-Koordinaten mit der Patientenliege 4 relativ zu der Therapiequelle 2 bewegt werden. Zur Lageerfassung der Therapiequelle 2 sowie der Ultraschallquelle 14 um die Schwenkwinkel α und β sind entsprechende Winkelsensoren vorgesehen. Das von der Ultraschallsonde 14 aufgenommene Ultraschallbild 20 ist ein Schnittbild durch den Körper des Patienten. Die Ebene des Ultraschallbildes 20 ist dabei um die Achse S um den Winkel a drehbar. Ferner kann sie gemeinsam mit der Therapiequelle 2 um den Winkel β entlang der bogenförmigen Bahn 10 verschwenken. In dem Ultraschallbild 20 ist hier schematisch ein Stein 22 gezeigt, welcher von dem Therapiefokus 16 beabstandet ist. Zur Behandlung des Steines ist es somit erforderlich, den Patienten 18 mit der Patientenliege 4 so zu bewegen, dass der Stein 22 mit dem Therapiefokus 16 zur Deckung kommt. Da das Ultraschallbild 20 um den Winkel a bzgl. der Achse S gedreht sein kann, erstreckt sich das Ultraschallbild 20 dabei nicht zwingend in der von den Koordinatenachsen X und Z der Antriebe aufgespannten Ebene. Es ist somit gegebenenfalls eine Verfahrbewegung aller drei Achsen X, Y und Z erforderlich. Dabei besteht die Schwierigkeit, den Stein 22 nicht aus dem Ultraschallbild 20 zu verlieren.

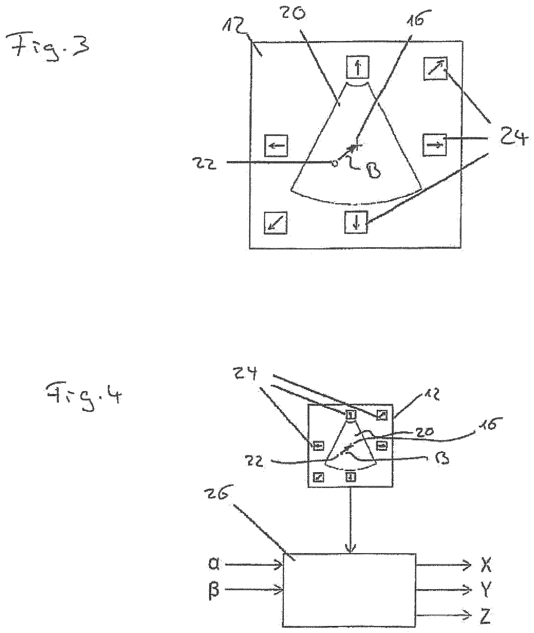

Fig. 3 zeigt beispielhaft die Darstellung des Ultraschallbildes 20 auf dem Ultraschallmonitor 12. In diesem Bild ist die Lage des Therapiefokus 16 gekennzeichnet und der Stein 22 erkennbar. Auf dem Monitor sind Richtungstasten 24 als berührungsempfindliche Felder dargestellt, mit welchen eine Bewegung der Patientenliege 4 veranlasst werden kann, sodass der Patient mit dem Stein 22 in den Therapiefokus 16 verfahren werden kann. Dazu ist in diesem Beispiel ein Bewegungspfad B in Form eines Vektors, welcher den Stein 22 und den Therapiefokus 16 in dem Ultraschallbild 20 verbindet, erforderlich. Es ist somit eine Verfahrbewegung genau in der aktuellen Ebene, d. h. Schnittebene des Ultraschallbildes 20 erforderlich. Die erforderlichen Bewegungen können durch die Richtungstasten 24 vom Benutzer am Ultraschallmonitor 12 ausgewählt werden.

Erfindungsgemäß ist nun eine Steuereinrichtung 26 vorgesehen, welche mit dem Ultraschallsteuergerät 11 verbunden ist und die Befehle der Richtungstasten 24 empfängt. Die Steuereinrichtung 26 erhält ferner über entsprechende Winkelsensoren als Eingangsgrößen die oben genannten Winkel a und β, um welche die Schnittebene des Ultraschallbildes 20 verschwenkt ist. Unter Berücksichtigung dieser Winkel transformiert die Steuereinrichtung 26 den Bewegungspfad bzw. Bewegungsvektor B in die drei Raumkoordinaten X, Y und Z der Antriebe der Patientenliege 4. Die Antriebe X, Y und Z erhalten dabei von der Steuereinrichtung 26 sowohl eine Weginformation bzgl. des Weges, welchen diese Antriebe entlang der jeweiligen Koordinatenachse zurücklegen müssen, sowie darüber hinaus eine an diese Wege angepasste Geschwindigkeitsinformation. D. h. die Steuereinrichtung 26 steuert die Antriebe der Achsen X, Y und Z so an, dass diese zeitgleich mit angepassten Geschwindigkeiten verfahren werden. Dabei wird der Antrieb, welcher den weitesten Weg zurückzulegen hat, am schnellsten und der Antrieb, welcher den kürzesten Weg zurückzulegen hat, am langsamsten angetrieben. Beträgt beispielsweise der Abstand zwischen Therapiefokus 16 und Stein 22 entlang der Z-Achse im Koordinatensystem der Patientenliege 4 drei cm, entlang der Y-Achse zwei cm und entlang der X-Achse ein cm, wird der Antrieb der Z-Achse mit der dreifachen Geschwindigkeit des Antriebes der X-Achse und der Antrieb der Y-Achse mit der doppelten Geschwindigkeit des Antriebes der X-Achse betrieben. So wird erreicht, dass diese drei Antriebe entlang der Achsen X, Y und Z bei zeitgleichem Antrieb eine resultierende Bewegung der Patientenliege 4 exakt entlang der Raumrichtung des Bewegungsvektors B durchführen. So wird erreicht, dass der Patient genau in der Ebene des Ultraschallbildes 20 bewegt wird und der Stein 22 somit bei der gesamten Bewegung im Ultraschallbild 20 verbleibt, sodass während der Bewegung eine visuelle Kontrolle durch den Benutzer möglich ist.

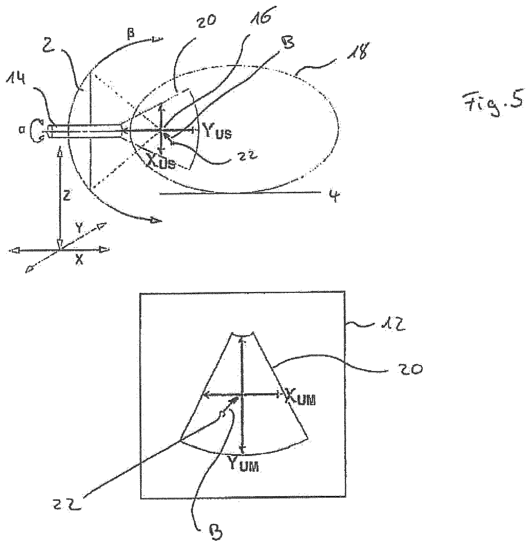

Wie anhand von Fig. 5 zu erkennen ist, erstreckt sich in dem zweidimensionalen Ultraschallbild 20 auf dem Ultraschallmonitor 12 die Y_{UM}-Koordinate in die gleiche Richtung wie die Yus-Achse der Ultraschallsonde 14. Dabei ist die Ultraschallsonde 14 um den Winkel a um die Yus-Achse drehbar. Die zu der Y_{UM}-Koordinate rechtwinklige Koordinate Y_{UM} im Ultraschallbild 20 geht stets in die gleiche Richtung wie die Xus-Achse der Ultraschallsonde 14. In der Steuereinrichtung 26 sind zur Transformation des Bewegungsvektors B in das kartesische Koordinatensystem mit den Achsen X, Y und Z der Patientenliege 4 die Winkel a und β zu kompensieren. So findet eine erste Vektorentransformation statt, in welcher der Winkel α kompensiert wird. In einer zweiten Vektorentransformation wird der Winkel β kompensiert. Wenn sich die Ultraschallsonde 14 entlang des Winkel β in ihrer in Fig. 5 gezeigten Mittelstellung befindet und sie sich in Richtung des Schwenkwinkels a in der in Fig. 5 gezeigten Mittelstellung befindet, erstreckt sich die Achse Yus des Ultraschallbildes 20 parallel zu der Bewegungsachse X der Patientenliege und die Achse Xus der Ultraschallsonde 14 erstreckt sich parallel zur Achse Z des Koordinatensystems der Antriebe der Patientenliege 4. Aus der Transformation des Winkels a in das Koordinatensystem X, Y und Z ergibt sich somit eine Bewegungsrichtung in der Y-Z-Ebene und aus der Transformation des Winkels β eine Bewegungsrichtung in der Z-X-Ebene dieses Koordinatensystems. So ergeben sich aus der Lage des Bewegungsvektors 22 entlang der Achsen Xus und Yus gemeinsam mit den Winkeln a und β Bewegungskoordinaten für die Antriebe in den Koordinatensystem X, Y und Z der Patientenliege 4. Um die Patientenliege 4 dann exakt entlang des Vektors 22 bewegen zu können, werden die Antriebe entlang der Achsen X, Y und Z gleichzeitig mit entsprechend angepassten Geschwindigkeiten verfahren.

Es ist zu verstehen, dass anstelle einer Bewegung der Patientenliege 4 auch die Therapiequelle 2 relativ zu der Patientenliege 4 entlang der Achsen X, Y und Z bewegt werden könnte. Auch wäre es beispielsweise denkbar, die Patientenliege 4 entlang nur einer oder zweier der Achsen X, Y und Z, beispielsweise entlang der Y und X zu bewegen, während die Therapiequelle entlang der anderen Achsen, beispielsweise entlang der Z-Achse bewegbar ist. Es kommt lediglich auf die Relativbewegung zwischen der Therapiequelle 2 und der Patientenliege 4 in dem kartesischen Koordinatensystem X, Y und Z an.

Um die von der Therapiequelle 2 erzeugten Stoßwellen in den Körper des Patienten 18 einkoppeln zu können, ist die Therapiequelle 2 mit einer wassergefüllten Vorlaufstrecke 28 versehen, welche mit einer Membran 30 an der Haut des Patienten 18 anliegt. Wenn nun die Patientenliege 4 entlang der Achse X relativ zu der Therapiequelle 2 verfahren wird, wird der Patient 18 so von der Therapiequelle 2 wegbewegt und die Membran 30 würde nicht mehr wie gewünscht flächig an der Oberfläche des Körpers des Patienten 18 anliegen. Um dies auszugleichen, ist eine Druckregelung in der Vorlaufstrecke 28 vorgesehen. Dazu ist in der Vorlaufstrecke 28 oder gekoppelt mit dieser ein Drucksensor 32 vorgesehen, welcher den Druck im Inneren der Vorlaufstrecke 28 erfasst. Über entsprechende Pumpen oder Ventile kann Flüssigkeit in die Vorlaufstrecke 28 eingeleitet oder aus dieser abgelassen werden. Dadurch ist es möglich, den Druck im Inneren der Vorlaufstrecke 28 im Wesentlichen konstant zu halten. Die Steuereinrichtung 26 ist dazu vorzugsweise so ausgebildet, dass, nachdem die Therapiequelle 2 mit der Vorlaufstrecke 28 und der Membran 30 in gewünschter Weise am Patienten 18 positioniert worden ist, der Druck im Inneren der Vorlaufstrecke 28 über den Drucksensor 32 erfasst wird und dann anschließend bei Bewegung des Patienten 18 auf der Patientenliege 4 relativ zu der Therapiequelle 2 der Druck in der Vorlaufstrecke 28 konstant oder in vordefinierten Grenzen gehalten wird, indem gegebenenfalls Flüssigkeit in die Vorlaufstrecke 28 eingeleitet bzw. eingepumpt wird oder bei zu hohem Druck abgelassen wird. So kann auch bei Bewegung des Patienten 18, sei es durch Verfahren der Patientenliege 4 oder aufgrund der Eigenbewegungen des Patienten 18 die Anlage der Membran 30 an der Haut des Patienten 18 stets in definierter Weise sichergestellt werden.

### Bezugszeichenliste

- 2: Therapiequelle
- 4: Patientenliege
- 6: Röntgen-C-Bogen
- 8: Röntgenbildverstärker
- 10: bogenförmige Bahn
- 11: Ultraschallsteuergerät
- 12: Ultraschallmonitor
- 14: Ultraschallsonde
- 16: Therapiefokus
- 18: Patient
- 20: Ultraschallbild
- 22: Stein
- 24: Richtungstasten
- 26: Steuereinrichtung
- 28: Vorlaufstrecke
- 30: Membran
- 32: Drucksensor

- S: Achse
- B: Bewegungspfad

- X_{US}, Y_{US}: Koordinaten der Ultraschallsonde 14
- X_{UM}, Y_{UM}: Koordinaten des Ultraschallbildes 20
- X, Y, Z: Koordinaten bzw. Verfahrachsen der Patientenliege 4 relativ zu der Therapiequelle 2
- α: Drehwinkel der Ultraschallsonde 14
- β: Schwenkwinkel der Therapiequelle 2

## Patentansprüche

1. Therapiesystem mit einer Strahlung oder Wellen aussendenden Therapiequelle (2), zumindest einer Ultraschallsonde (14), einer Patientenliege sowie einem Mehr-Achsen-Positioniersystem (X, Y, Z) mit mehreren Antrieben, mittels welchen die Patientenliege (4) und die Therapiequelle (2) in allen drei Raumrichtungen relativ zueinander bewegbar sind,
und mit einer Steuereinrichtung (26), welche mit der Ultraschallsonde (14) derart gekoppelt und derart ausgebildet ist, dass sie in zumindest einem Betriebsmodus die Antriebe (X, Y, Z) des Mehr-Achsen-Positioniersystems (X, Y, Z) in einer Weise gleichzeitig so ansteuert, dass diese zeitgleich mit individuell von der Steuereinrichtung (26) definierten Geschwindigkeiten verfahren werden, und dass die Patientenliege relativ zu der Therapiequelle entlang eines auswählbaren Bewegungspfades (B) bewegt wird, welcher innerhalb der Ebene eines aktuell von der Ultraschallsonde (14) aufgenommenen Ultraschallbildes (20) liegt, wobei die Steuereinrichtung derart ausgestaltet ist, dass die Geschwindigkeiten, mit welchen die Antriebe (X, Y, Z) verfahren, von der Steuereinrichtung (26) derart festgelegt werden, dass das Verhältnis der Verfahrgeschwindigkeiten zueinander dem Verhältnis der von den Antrieben (X, Y, Z) zum Verfahren entlang des Bewegungspfades (B) zurückzulegenden Wege entspricht.

2. Therapiesystem nach Anspruch 1, **dadurch gekennzeichnet, dass** die Steuereinrichtung (26) derart ausgebildet ist, dass in dem Ultraschallbild (20) als Bewegungspfad (B) ein Vektor auswählbar ist.

3. Therapiesystem nach einem der vorangehenden Ansprüche, **dadurch gekennzeichnet, dass** drei Antriebe (X, Y, Z) vorgesehen sind, welche eine Bewegung entlang dreier zueinander normaler Achsen ermöglichen.

4. Therapiesystem nach einem der vorangehenden Ansprüche, **dadurch gekennzeichnet, dass** die Ultraschallsonde (14) derart in die Therapiequelle (2) integriert ist, dass ein Therapiefokus (16) der Therapiequelle (2) in dem von der Ultraschallsonde (14) aufgenommen Ultraschallbild (20) gelegen ist.

5. Therapiesystem nach Anspruch 4, **dadurch gekennzeichnet, dass** die Ultraschallsonde (14) um eine sich durch den Therapiefokus (16) erstreckende Achse (S) relativ zu der Therapiequelle (2) drehbar ist, wobei ein mit der Steuereinrichtung (26) verbundenes erstes Winkelmesssystem zum Erfassen des Drehwinkels (α) zwischen der Ultraschallsonde (14) und der Therapiequelle (2) vorhanden ist.

6. Therapiesystem nach einem der vorangehenden Ansprüche, bei welchem die Therapiequelle (2) relativ zu der Patientenliege (4) entlang einer bogenförmigen Bahn (10) schwenkbar ist, wobei ein mit der Steuereinrichtung (26) verbundenes zweites Winkelmesssystem zum Erfassen des Schwenkwinkels (β) zwischen der Therapiequelle (2) und der Patientenliege (4) vorhanden ist.

7. Therapiesystem nach einem der vorangehenden Ansprüche, **dadurch gekennzeichnet, dass** die Steuereinrichtung (26) derart ausgebildet ist, dass sie in dem zumindest einen Betriebsmodus die Antriebe (X, Y, Z) derart ansteuert, dass eine Relativbewegung zwischen der Patientenliege (4) und der Therapiequelle (2) ausschließlich entlang von Bewegungspfaden (B) möglich ist, welche innerhalb der Ebene des Ultraschallbildes (20) gelegen sind.

8. Therapiesystem nach einem der vorangehenden Ansprüche, **dadurch gekennzeichnet, dass** die Steuereinrichtung (26) derart ausgebildet ist, dass von einem Benutzer der Bewegungspfad (B) für eine gewünschte Relativbewegung zwischen Patientenliege (4) und Therapiequelle (2) innerhalb der Ebene des aktuellen Ultraschallbildes (20) auswählbar ist.

9. Therapiesystem nach Anspruch 8, **dadurch gekennzeichnet, dass** auf einem das Ultraschalbild (20) darstellenden Bildschirm (12) oder seitlich eines das Ultraschallbild (20) darstellenden Bildschirmes (12) Tasten (24) zur Auswahl einer den Bewegungspfad (B) definierenden Bewegungsrichtung angeordnet sind.

10. Therapiesystem nach Anspruchs oder 9, **dadurch gekennzeichnet, dass** die Steuereinrichtung (26) derart ausgebildet ist, dass sie die räumliche Lage des ausgewählten Pfades (B) in das Koordinatensystem des Mehr-Achsen-Positioniersystems transformiert und die Antriebe (X, Y, Z) derart ansteuert, dass eine Bewegung entlang dem transformierten Pfad erfolgt.

11. Therapiesystem nach einem der vorangehenden Ansprüche, **gekennzeichnet durch** ein Membrandruckregelsystem, welches derart ausgebildet ist, dass es einen Anlagedruck einer dem Patienten zugewandten Außenfläche (30) der Therapiequelle (2) durch Druckbeaufschlagung oder Druckentlastung einer in der Therapiequelle (2) ausgebildeten Vorlaufstrecke (28) konstant hält

12. Verfahren zur Positionierung einer Patientenliege relativ zu einem Therapiequelle (2) anhand eines aktuellen Ultraschallbildes (20), bei welchem
in dem aktuellen Ultraschallbild (20) in der Ebene des Ultraschallbildes (20) ein Bewegungspfad (B) definiert wird, entlang dem die Patientenliege (4) relativ zu der Therapiequelle (2) verfahren werden soll,
anschließend Antriebe (X, Y, Z), mittels welchen die Patientenliege (4) relativ zu der Therapiequelle (2) bewegbar ist, gleichzeitig so angetrieben werden, dass eine Bewegung der Patientenliege (4) relativ zu der Therapiequelle (2) dem definierten Bewegungspfad (B) folgt, wobei die Verfahrgeschwindigkeiten der einzelnen Antriebe (X, Y, Z) so eingestellt werden, dass deren Verhältnis zueinander dem Verhältnis der Wege zueinander entspricht, welche von den einzelnen Antrieben (X, Y, Z) zum Verfahren entlang des Bewegungspfades (B) zurückzulegenden sind.

13. Verfahren nach Anspruch 12, **dadurch gekennzeichnet, dass** der Bewegungspfad (B) ein Vektor ist.

14. Verfahren nach Anspruch 12 oder 13, **dadurch gekennzeichnet, dass** die räumliche Lage des Bewegungspfades (B) unter Berücksichtigung der aktuellen räumlichen Lage der Ebene des Ultraschallbildes (20) in ein Koordinatensystem der Bewegungsachsen, entlang derer die Patientenliege relativ zu der Therapiequelle (2) bewegt wird, transformiert wird und anschließend die Antriebe (X, Y, Z) gleichzeitig so angesteuert werden, dass eine Bewegung entlang dieses transformierten Bewegungspfades (B) erfolgt.

## Claims

1. A therapy system with a therapy source (2) emitting radiation or waves, at least one ultrasound probe (14), a patient rest as well as a multi-axis positioning system (X, Y, Z) with several drives, by way of which the patient rest (4) and the therapy source (2) can be moved relative to one another in all three spatial directions,
and with a
control device (26) which is coupled to the ultrasound probe (14) and designed, in such a manner that in at least one operating mode it simultaneously activates the drives (X, Y, Z) of the multi-axis positioning system (X, Y, Z) in a manner such that these are displaced simultaneously at speeds individually defined by the control device (26), and that the patient rest is moved relative to the therapy source along a selectable movement path (B) which lies within the plane of an ultrasound picture (20) which is currently recorded by the ultrasound probe (14), wherein the control device is designed in a manner such that the speeds, with which the drives (X, Y, Z) are displaced, are specified by the control device (26) in a manner such that the ratio of the displacement speeds to one another corresponds to the ratio of the distances which are to be covered by the drives (X, Y, Z) for displacing along the movement path (B).

2. A therapy system according to claim 1, **characterised in that** the control device (26) is designed in a manner such that a vector can be selected in the ultrasound picture (20) as a movement path (B).

3. A therapy system according to one of the preceding claims, **characterised in that** three drives (X, Y, Z) are provided and these permit a movement along three axes which are normal to one another.

4. A therapy system according to one of the preceding claims, **characterised in that** the ultrasound probe (14) is integrated into the therapy source (2) in a manner such that a therapy focus (16) of the therapy source (2) is situated in the ultrasound picture (20) which is recorded by the ultrasound probe (14).

5. A therapy system according to claim 4, **characterised in that** the ultrasound probe (14) is rotatable relative to the therapy source (2), about an axis (S) extending through the therapy focus (16), wherein a first angular measurement system which is connected to the control device (26) is present for detecting the rotation angle (a) between the ultrasound probe (14) and the therapy source (2).

6. A therapy system according to one of the preceding claims, **characterised in that** the therapy source (2) is pivotable relative to the patient rest (4) along an arcuate path (10), wherein a second angular measurement system which is connected to the control device (26) is present for detecting the pivot angle (β) between the therapy source (2) and the patient rest (4).

7. A therapy system according to one of the preceding claims, **characterised in that** the control device (26) is designed in a manner such that in the at least one operating mode, it activates the drives (X, Y, Z) in a manner such that a relative moment between the patient rest (4) and the therapy source (2) is possible exclusively along movement paths (B) which are situated within the plane of the ultrasound picture (20).

8. A therapy system according to one of the preceding claims, **characterised in that** the control device (26) is designed in a manner such that the movement path (B) for a desired relative movement between the patient rest (4) and the therapy source (2) within the plane of the current ultrasound picture (20) can be selected by the user.

9. A therapy system according to claim 8, **characterised in that** keys (24) for the selection of a movement direction defining the movement path (B) are arranged on a screen (12) representing the ultrasound picture (20) or laterally of a screen (12) representing the ultrasound picture (20).

10. A therapy system according to claim 8 or 9, **characterised in that** the control device (26) is designed in a manner such that it transforms the spatial position of the selected path (B) into the coordinate system of the multi-axis positioning system, and activates the drives (X, Y, Z) in a manner such that a movement along the transformed path is effected.

11. A therapy system according to one of the preceding claims, **characterised by** a membrane pressure control system which is designed in a manner such that it keeps constant a contact pressure of an outer surface (30) of the therapy source (2) which faces the patient, by way of pressure subjection or pressure relief of a transmission path (28) formed in the therapy source (2).

12. A method for positioning a patient rest relative to a therapy source (2) by way of a current ultrasound picture (20),
with which
a movement path (B), along which the patient rest (4) is to be displaced relative to the therapy source (2) is defined in the current ultrasound picture (20) in the plane of the ultrasound picture (20),
drives (X, Y, Z), by way of which the patient rest (4) can be moved relative to the therapy source (2), are subsequently driven simultaneously such that a movement of the patient rest (4) relative to the therapy source (2) follows the defined movement path (B), wherein the displacement speeds of the individual drives (X, Y, Z) are set such that their ratio to one another corresponds to the ratio to one another of the distances which are to be covered by the individual drives (X, Y, Z) for displacing along the movement path (B).

13. A method according to claim 12, **characterised in that** the movement path (B) is a vector.

14. A method according to claim 12 or 13, **characterised in that** the spatial position of the movement path (B) is transformed into a coordinate system of the movement axes, along which the patient rest is moved relative to the therapy source (2), whilst taking into account the current spatial position of the plane of the ultrasound picture (20), and subsequently the drives (X, Y, Z) are simultaneously activated such that a movement is effected along this transformed movement path (B).

## Revendications

1. Système thérapeutique avec une source thérapeutique (2) émettant un rayonnement ou des ondes, au moins une sonde ultrasons (14), une table médicale ainsi qu'un système de positionnement multiaxes (X, Y, Z) avec plusieurs entraînements à l'aide duquel la table médicale (4) et la source thérapeutique (2) peuvent être déplacées l'une par rapport à l'autre dans les trois directions de l'espace,
et avec un dispositif de commande (26) qui est relié à la sonde ultrasons (14) de façon telle et qui est configuré de façon telle que, dans au moins un mode de fonctionnement, il asservit les entraînements (X, Y, Z) du système de positionnement multiaxes (X, Y, Z) simultanément d'une manière telle sorte qu'ils se déplacent simultanément à des vitesses définies individuellement par le dispositif de commande (26), et que la table médicale soit déplacée par rapport à la source thérapeutique le long d'un trajet de déplacement (B) choisissable qui est situé à l'intérieur du plan d'une image ultrasons (20) actuellement prise par la sonde ultrasons (14), le dispositif de commande étant configuré de façon que les vitesses avec lesquelles les entraînements (X, Y, Z) se déplacent, soient déterminées par le dispositif de commande (26) de façon telle que le rapport entre les vitesses de déplacement corresponde au rapport entre les chemins parcourus par les entraînements (X, Y, Z) pour le déplacement le long du trajet de déplacement (B).

2. Système thérapeutique selon la revendication 1, **caractérisé en ce que** le dispositif de commande (26) est configuré de façon telle que puisse être choisi, dans l'image ultrasons (20), comme trajet de déplacement (B), un vecteur.

3. Système thérapeutique selon l'une des revendications précédentes, **caractérisé en ce que** trois entraînements (X, Y, Z) sont prévus qui permettent un déplacement le long de trois axes perpendiculaires les uns aux autres.

4. Système thérapeutique selon l'une des revendications précédentes, **caractérisé en ce que** la sonde ultrasons (14) est intégrée dans la source thérapeutique (2) de façon qu'un point de focalisation thérapeutique (16) de la source thérapeutique (2) soit situé dans l'image ultrasons (20) prise par la sonde ultrasons (14).

5. Système thérapeutique selon la revendication 4, **caractérisé en ce que** la sonde ultrasons (14) est rotative par rapport à la source thérapeutique (2) autour d'un axe (S) s'étendant par le point de focalisation thérapeutique (16), un premier système de mesure d'angle, relié au dispositif de commande (26), étant présent pour saisir un angle de rotation (a) entre la sonde ultrasons (14) et la source thérapeutique (2).

6. Système thérapeutique selon l'une des revendications précédentes, dans lequel la source thérapeutique (2) est pivotante, par rapport à la table médicale (4), le long d'un trajet (10) ayant la forme d'un arc, un deuxième système de mesure d'angle, relié au dispositif de commande (26), étant présent pour saisir un angle de pivotement (β) entre la sonde ultrasons (14) et la source thérapeutique (2).

7. Système thérapeutique selon l'une des revendications précédentes, **caractérisé en ce que** le dispositif de commande (26) est configuré de façon qu'il asservisse les entraînements (X, Y, Z) dans ledit au moins un mode de fonctionnement de façon telle qu'un mouvement relatif entre la table médicale (4) et la source thérapeutique (2) soit possible exclusivement le long de trajets de déplacements (B) qui sont situés à l'intérieur du plan de l'image ultrasons (20).

8. Système thérapeutique selon l'une des revendications précédentes, **caractérisé en ce que** le dispositif de commande (26) est configuré de façon que le trajet de déplacement (B) pour un mouvement relatif souhaité entre la table médicale (4) et la source thérapeutique (2) puisse être sélectionné par un utilisateur à l'intérieur de l'image ultrasons (20) actuelle.

9. Système thérapeutique selon la revendication 8, **caractérisé en ce que** des touches (24) pour la sélection d'une direction de mouvement définissant le trajet de déplacement (B) sont disposées sur un écran (12) représentant l'image ultrasons (20) ou à côté d'un écran (12) représentant l'image ultrasons (20).

10. Système thérapeutique selon la revendication 8 ou 9, **caractérisé en ce que** le dispositif de commande (26) est configuré de façon qu'il transforme la position dans l'espace du trajet (B) choisi, dans le système de coordonnées du système de positionnement multiaxes et qu'il asservisse les entraînements (X, Y, Z) de façon qu'un mouvement soit effectué le long du trajet transformé.

11. Système thérapeutique selon l'une des revendications précédentes, **caractérisé par** un système de régulation de pression de membrane qui est configuré de façon qu'il maintienne constante une pression d'appui d'une surface extérieure (30) de la source thérapeutique (2), orientée vers le patient, en augmentant ou réduisant la pression d'un trajet précurseur (28) formée dans la source thérapeutique (2).

12. Procédé pour positionner une table médicale par rapport à une source thérapeutique (2) sur la base d'une image ultrasons (20), selon lequel procédé
dans l'image ultrasons (20) actuelle, un trajet de déplacement (B) est défini dans le plan de l'image ultrasons (20), le long duquel la table médicale (4) doit être déplacée par rapport à la source thérapeutique (2),
ensuite, des entraînements (X, Y, Z) à l'aide desquels la table médicale (4) est déplaçable par rapport à la source thérapeutique (2), sont entraînés simultanément de façon qu'il en résulte un mouvement de la table médicale (4) par rapport à la source thérapeutique le long du trajet de déplacement (B) défini, les vitesses de déplacement des différents entraînements (X, Y, Z) étant ajustées de façon que le rapport entre elles corresponde au rapport entre les chemins qui sont à parcourir par les entraînements (X, Y, Z) pour le déplacement le long du trajet de déplacement (B).

13. Procédé selon la revendication 12, **caractérisé en ce que** le trajet de déplacement (B) est un vecteur.

14. Procédé selon la revendication 12 ou 13, **caractérisé en ce que** la position dans l'espace du trajet (B) est transformée, en tenant compte de la position actuelle dans l'espace du plan de l'image ultrasons (20), en un système de coordonnées des axes de déplacement le long desquels la table médicale est déplacée par rapport à la source thérapeutique (2), et que, ensuite, les entraînements (X, Y, Z) sont asservis simultanément de façon qu'un mouvement soit effectué le long du trajet de déplacement (B) transformé.
